# EUROPÄISCHE PATENTSCHRIFT

(11) **EP 3 030 217 B1**
(45) Veröffentlichungstag und Bekanntmachung des Hinweises auf die Patenterteilung: **21.11.2018**
(21) Anmeldenummer: 14755012.3
(22) Anmeldetag: 23.07.2014
(51) Int. Cl.: A61K 8/31, A61K 8/34, A61Q 5/10, A61K 8/86

(54) **MEHRKOMPONENTEN-VERPACKUNGSEINHEIT ZUM OXIDATIVEN FÄRBEN VON KERATINISCHEN FASERN MIT REDUZIERTEM AMMONIAK-GERUCH**
MULTI-COMPONENT PACKAGING UNIT FOR OXIDATIVELY DYEING KERATIN FIBERS, HAVING REDUCED AMMONIA ODOR
UNITÉ D'EMBALLAGE MULTICOMPOSANTE POUR LA COLORATION DE FIBRES KERATINIQUES PAR OXIDATION AYANT UNE ODEUR D'AMMONIA REDUITE

(30) Priorität: 07.08.2013 DE 102013215583
(43) Veröffentlichungstag der Anmeldung: 15.06.2016
(73) Patentinhaber: Henkel AG & Co. KGaA, 40589 Düsseldorf (DE)
(72) Erfinder: NEUBA, Constanze, 41516 Grevenbroich (DE); MÜLLER, Burkhard, 40221 Düsseldorf (DE)
(86) Internationale Anmeldenummer: PCT/DE2014/200343
(87) Internationale Veröffentlichungsnummer: WO 2015/018412

(56) Entgegenhaltungen:
- EP-A1- 2 915 522
- JP-A- 2008 019 220
- JP-A- 2008 290 949
- JP-A- 2013 112 641

## Beschreibung

Die vorliegende Erfindung liegt auf dem Gebiet der Kosmetik und betrifft Mehrkomponenten-Verpackungseinheiten (Kit-of-parts) zum oxidativen Färben von keratinischen Fasern, die mindestens zwei getrennt voneinander konfektionierte Zubereitungen (A) und (B) umfassen. Der Einsatz der aus (A) und (B) hergestellten Anwendungsmischung ermöglicht die oxidative Färbung von Keratinfasern und hat einen verringerten Ammoniak-Geruch. Ein weiterer Gegenstand der vorliegenden Erfindung ist ein Verfahren zum oxidativen Färben von Keratinfasern.

Zur Bereitstellung farbverändernder kosmetischer Mittel, insbesondere für keratinische Fasern, wie beispielsweise Haare, kennt der Fachmann je nach Anforderung an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch hervorragende, lang anhaltende Färbeergebnisse aus. Das im Oxidationsfärbemittel enthaltene Oxidationsmittel (d.h. Wasserstoffperoxid) initiiert hierbei nicht nur die Bildung der Farbstoffe, sondern es zerstört oxidativ auch die haareigenen Farbpigmente (Melanine), so dass im Fall der oxidativen Färbung auch gleichzeitig eine aufhellende Färbung möglich wird. Um eine zufriedenstellende Färbe- und Aufhellleistung zu entfalten, benötigen oxidative Färbemittel bei der Anwendung in der Regel einen alkalischen pH-Wert; insbesondere bei pH-Werten zwischen 8,5 und 10,5 werden optimale Ergebnisse erzielt.

Zur Einstellung dieser pH-Werte ist bis zum heutigen Zeitpunkt Ammoniak das Alkalisierungsmittel der Wahl. Mit Ammoniak lässt sich nicht nur der für die Farbstoffbildung notwendige pH-Bereich einstellen, sondern Ammoniak sorgt auch in stärkerem Maß als alle anderen bekannten Alkalisierungsmittel für eine Quellung der Haare. Gleichzeitig wirkt Ammoniak - ebenfalls in stärkerem Ausmaß als alle anderen handelsüblichen Alkalisierungsmittel - als Penetrations- bzw. Penetrationshilfsmittel.

Aus diesen Gründen werden bei Einsatz von Ammoniak in oxidativen Färbemitteln im Vergleich zu anderen Alkalisierungsmitteln (wie beispielsweise Kalium- oder Natriumhydroxid, Alkanolaminen oder Carbonaten wie Natriumcarbonat oder Kaliumcarbonat) intensivere Färbungen und signifikant bessere Grauabdeckungen erhalten.

Bedingt durch die von Anfang an höheren Farbintensitäten sind auch die Echtheitseigenschaften der mit Hilfe von Ammoniak erzeugten Haarfärbungen besser. Insbesondere besitzen gefärbte Haare dann die besten Waschechheiten, wenn Ammoniak als Alkalisierungsmittel gewählt wurde.

Die mit dem Einsatz von Ammoniak verbundenen anwendungstechnischen Vorteile sind so vielfältig, dass Ammoniak trotz seines unangenehmen, stechenden Geruchs in einer Vielzahl handelsüblicher oxidativer Färbemittel verwendet wird.

Aus der Literatur sind bereits umfangreiche Bestrebungen zur Reduzierung des Ammoniak-Geruchs bekannt. Hierbei bestehen zur Minimierung des Geruchs drei prinzipielle Möglichkeiten: Als erste Möglichkeit nennt die Literatur die Variation des Alkalisierungsmittels und damit den teilweisen oder vollständigen Ersatz von Ammoniak durch geruchslose Alternativen.

So existiert beispielsweise eine Vielzahl von Rezepturen, in welchen ein Gemisch von Ammoniak und Monoethanolamin oder ausschließlich Monoethanolamin als Alkalisierungsmittel einsetzt wird. Eine Reduktion des Ammoniak-Gehaltes hat jedoch generell eine schlechtere Penetration der Farbstoffe in das Haar hinein zur Folge, was sich wie zuvor beschrieben insbesondere in schlechteren Grauabdeckungen und einer schlechteren Waschechtheiten niederschlägt. Wenn die Entwicklung von besonders haltbaren Nuancen im Fokus steht, ist der Einsatz von Monoethanolamin deshalb keine Option.

Die WO 2006060570 und WO 2006060565 schlagen für die Bereitstellung von oxidativen Färbemitteln mit geringer Geruchsbelastung den Einsatz von Carbonaten oder Carbonatquellen als Alkalisierungsmittel vor. Es ist jedoch ebenfalls literaturbekannt, dass Carbonate in Kombination mit Oxidationsmitteln die Haare in stärkerem Ausmaß schädigen können. Die durch die Carbonate hervorgerufene zusätzliche Schädigung des Haares mag bei Anwendung des Färbemittels auf unbehandeltem bzw. ungeschädigtem Haar wenig problematisch sein, kann sich jedoch bei Personen, die ihr Haar regelmäßig färben bzw. blondieren, zu schweren, kumulativen Schäden aufsummieren. Wird eine stärkere Aufhellung und/oder regelmäßige Färbung gewünscht, stellt der Einsatz von Carbonaten daher ebenfalls keine gangbare Alternative dar.

Eine zweite prinzipielle Möglichkeit zur Reduktion des Ammoniak-Geruchs besteht im Zusatz von speziellen Parfumstoffen, welche den Ammoniak-Geruch überdecken sollen. Dieser Weg wird beispielsweise in der WO 2005/110499 beschritten. Parfumstoffe können jedoch unter den alkalischen Lagerbedingungen instabil sein, so dass die Gefahr besteht, dass die Dufstoffe während der Lagerung abgebaut oder strukturell verändert werden, was sich auch in einer unvorhersehbaren Änderung des Geruchs niederschlägt. Da entsprechende Veränderungen oftmals erst nach mehreren Monaten oder sogar Jahren wahrnehmbar werden, wird der Einsatz von neuen bzw. unbekannten Parfums als problematisch eingestuft.

Eine dritte prinzipielle Möglichkeit zur Reduktion des Ammoniakgeruchs besteht in einer Optimierung der Formulierung. Hierbei gilt es, die Träger-Bestandteile der Formulierung so auszuwählen, dass diese einen optimalen Rückhalt des Ammoniaks in der Formulierung gewährleisten und auf diese Weise seinen Geruch minimieren. Es ist jedoch ebenfalls bekannt, dass die Formulierung, die in ihr enthaltenen Fettstoffe, ihre Emulgatoren, Tenside und auch die Viskosität wesentlichen Einfluss auf die Färbeleistung nehmen. Bei der Modifikation der Formulierung muss eine Verschlechterung der Färbeleistung daher auf jeden Fall vermieden werden.

Beispielsweise schlägt die JP 2007191459 zur Reduktion des Ammoniak-Geruchs in Haarfärbemitteln den Einsatz von kationischen Tensiden, Phosphateestern und aliphatischen Alkoholen vor.

Die JP 2003040750 offenbart, dass der Ammoniak-Geruch in Blondiermittel dann besonders gering ist, wenn den Mitteln mindestens 5 % einer kristallinen Komponente zugesetzt wird.

JP 2008290949 beschreibt Haarfärbe- und Bleichmittel, die vor der Anwendung durch Vermischen von zwei Komponenten hergestellt werden, wobei die Anwendungsmischung ein Alkalisierungsmittel, einen Polyoxyethylen-alkyl-ether, ein Oxidationsmittel und einen Fettalkohol enthält.

Insbesondere die dauerhafte Geruchsminimierung über die gesamte Anwendungsdauer ist nur sehr schwierig zu erreichen. Die Zeitspanne, innerhalb der sich der Anwender von Haarfarben in Kontakt mit dem Färbemittel befindet, reicht von der Herstellung der Anwendungsmischung über deren Auftragung auf die Haare und den Zeitraum des Einwirkens bis zum Auswaschen der Formulierung. Bei üblichen Einwirkzeiten von 30 bis 45 Minuten kann der gesamte Prozess bis zu 90 Minuten, im Höchstfall bis zu zwei Stunden Zeit in Anspruch nehmen. Eine geruchliche Abdeckung des Ammoniaks, die über diesen gesamten Zeitraum wirksam ist, stellt die höchste Herausforderung dar. Gerade auf diesem Feld besteht noch starker Optimierungsbedarf, und eine optimale Möglichkeit zur dauerhaften Reduktion des Ammoniak-Geruchs ist aus dem Stand der Technik bislang nicht bekannt.

Aufgabe der vorliegenden Erfindung war daher die Bereitstellung von Mitteln zum oxidativen Färben und/oder Aufhellen von Haaren mit reduziertem Ammoniakgeruch. Hierbei sollen die Mittel keine Einbußen in ihrer färberischen Leistung, insbesondere bei ihrer Grauabdeckung und ihrer Waschechtheit, aufweisen. Darüber hinaus soll die Anwendung der Mittel nicht mit einer höheren Haarschädigung verbunden sein, sich einfach anwenden lassen und lagerstabil sein.

Es konnte nun gefunden werden, dass die geruchliche Belästigung während des Färbevorgangs minimiert werden kann, wenn die Mittel in Form einer Mehrkomponenten-Verpackungseinheit (Kit-of-parts) bereitgestellt werden, die mindestens zwei getrennt voneinander verpackte Zubereitungen (A) und (B) umfasst, wobei deren Komponente (B) als spezielle Inhaltsstoffe die Kombination aus langkettigen Fettalkoholen, Kohlenwasserstoffen und ethoxylierten Fettalkoholen enthält.

Ein erster Gegenstand der vorliegenden Erfindung ist eine Mehrkomponenten-Verpackungseinheit (Kit-of-parts) zum oxidativen Färben von keratinischen Fasern, umfassend zwei getrennt voneinander verpackte Zubereitungen (A) und (B), wobei (a) die Zubereitung (A) in einem kosmetischen Träger (a1) mindestens ein Oxidationsfarbstoffvorprodukt und (a2) Ammoniak enthält, und (b) die Zubereitung (B) in einem kosmetischen Träger (b1) Wasserstoffperoxid, (b2) mindestens einen Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol), (b3) mindestens einen Kohlenwasserstoff mit 8 bis 80 C-Atomen und (b4) mindestens einen ethoxylierten Fettalkohol der Formel (I), 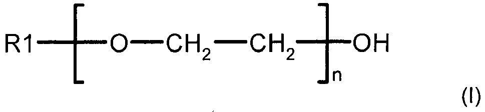 worin R1 für eine unverzweigte oder verzweigte, gesättigte oder ungesättigte C₁₂-C₂₈-Alkylgruppe steht und n für eine ganze Zahl von 80 bis 120 steht, enthält. Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare._Die Zubereitungen (A) und (B) enthalten die erfindungswesentlichen Bestandteile jeweils in einem kosmetischen Träger, bevorzugt in einem geeigneten wässrigen, alkoholischen oder wässrig-alkoholischen Träger. Zum Zwecke der Oxidationsfärbung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein. Das erfindungsgemäße Oxidationsfärbemittel umfasst mindestens zwei getrennt voneinander verpackten Zubereitungen (A) und (B). Kurz vor der Anwendung werden beide Zubereitungen miteinander vermischt. Auf diese Weise wird das anwendungsbereite Färbemittel hergestellt, das auf die Keratinsfasern appliziert wird._Die getrennt voneinander verpackten Zubereitungen (A) und (B) können in separaten Containern bereitgestellt werden, die sich zusammen in einer Umverpackung (z.B. einer Cartonage oder einem Carton befinden). Ebenso erfindungsgemäß ist es jedoch auch, die Zubereitungen (A) und (B) in zwei separaten Containern bereit zustellen, die getrennt voneinander (beispielsweise innerhalb einer Produktserie) käuflich erwerblich sind und die vor der Anwendung miteinander vermischt werden.

Weiterhin ist es ebenfalls möglich und erfindungsgemäß, dass das anwendungsbereite Färbemittel vor der Anwendung aus drei getrennt voneinander konfektionierten Komponenten hergestellt wird. In diesem Fall werden vor der Anwendung die drei Komponenten (A), (B) und (C) miteinander vermischt und die nach dem Vermischen der drei Zubereitungen resultierende Anwendungsmischung auf die Keratinfasern appliziert. Auch die drei Komponenten (A), (B) und (C) können in drei separaten Containern bereitgestellt werden, die sich innerhalb einer gemeinsamen Umverpackung befinden, oder aber komplett separariert (z.B. innerhalb einer Produktserie) dem Verbraucher zur Verfügung gestellt werden. Es ist bevorzugt, wenn das anwendungsbereite Oxidationsfärbemittel durch Vermischen von nur zwei Zubereitungen (A) und (B) hergestellt wird. Bei der in der Mehrkomponenten-Verpackungseinheit (Kit-of-parts) enthaltenen Zubereitung (A) handelt es sich um die Färbecreme, die in einem kosmetischen Träger mindestens ein Oxidationsfarbstoffvorprodukt (a1) und den für die Quellung, Penetration und Alkalisierung notwendigen Ammoniak (a2) enthält.

Unter die Oxidationsfarbstoffvorprodukte (a1) fallen Oxidationsfarbstoffvorprodukte vom Entwickler-Typ und vom Kuppler-Typ. Bevorzugte Oxidationsfarbstoffvorprodukte vom Entwickler-Typ sind ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)-1H-pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders bevorzugte Oxidationsfarbstoffvorprodukte vom Entwickler-Typ sind ausgewählt aus der Gruppe p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)-methan, p-Aminophenol, 4-Amino-3-methylphenol, 4,5-Diamino-1-(2-hydroxyethyl)-1H-pyrazol, 2,4,5,6-Tetraaminopyrimidin, 4-Hydroxy-2,5,6-triaminopyrimidin, 2-Hydroxy-4,5,6-triaminopyrimidin, sowie deren physiologisch verträglichen Salzen.

Kupplerkomponenten bilden im Rahmen der oxidativen Färbung allein keine signifikante Färbung aus, sondern benötigen stets die Gegenwart von Entwicklerkomponenten. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus.

Besonders geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diamino-phenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die Oxidationsfarbstoffvorprodukte (Entwickler und Kuppler) können in der Zubereitung (A) in einer Gesamtmenge von 0,01 bis 6,5 Gew.-%, bevorzugt von 0,05 bis 5,5 Gew.-%, weiter bevorzugt von 0,1 bis 4,5 Gew.-% und besonders bevorzugt von 0,3 bis 4,0 Gew.-% - bezogen auf das Gesamtgewicht der Zubereitung (A) - enthalten sein.

Zusätzlich zu den Oxidatonsfarbstoffvorprodukten kann die Zubereitung (A) auch einen oder mehrere direktziehende Farbstoffe enthalten. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen.

Besonders bevorzugt handelt es sich hierbei um einen oder mehrere nichtionische direktziehende Farbstoffe aus der Gruppe, HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)-amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Als direktziehende Farbstoffe können auch eine oder mehrere Verbindungen enthalten sein, die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannt sind.

Geeigenete kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls geeignete kationische direktziehende Farbstoffe.

Zusätzlich in der Zubereitung (A) vorhandene direktziehende Farbstoffe können in einer Gesamtmenge von 0,001 bis 4,0 Gew.-%, bevorzugt von 0,01 bis 3,0 Gew.-%, weiter bevorzugt von 0,05 bis 2,0 Gew.-% und ganz besonders bevorzugt von 0,1 bis 1,5 Gew.-% - bezogen auf das Gesamtgewicht der Zubereitung (A) - eingesetzt werden.

Als zweite wesentliche Komponente enthält die Zubereitung (A) Ammoniak (a2).

Üblicherweise wird Ammoniak (NH₃) in Form seiner wässrigen Lösung eingesetzt, in welcher es in Form von Ammoniumhydroxid (NH₄OH) vorliegt. Wässrige Ammoniak-Lösungen enthalten Ammoniak (NH₃) oft in Konzentrationen zwischen 10 Gew.-% und 32 Gew.-%. Bevorzugt ist hierbei der Einsatz einer wässrigen Ammoniak-Lösung, die 25 Gew.-% Ammoniak (NH₃) enthält.

Da Ammoniak vornehmlich als Alkalisierungsmittel eingesetzt wird, hängen dessen Einsatzmengen davon ab, welchen pH-Wert das anwendungsbereite Oxidationsfärbemittel besitzen soll. Dem Fachmann ist bekannt, dass mit steigender Ammoniak-Menge auch der pH-Wert der Zubereitung (A) steigt. Bei Vermischen der Zubereitungen (A) und (B) bestimmt der Ammoniak-Gehalt so auch den pH-Wert der Anwendungmischung mit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, dass die Einsatzmenge an Ammoniak so gewählt wird, dass der pH-Wert des anwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Zur Einstellung dieser pH-Werte können - in Abhängigkeit von weiteren, gegebenenfalls in der Anwendungmischung enthaltenenen Säuren, Puffern oder anderen Alkalisierungsmitteln - Ammoniakmengen von 0,1 bis 6,0 Gew.-%, bevorzugt von 0,3 bis 4,5 Gew.-%, weiter bevorzugt von 0,4 bis 3,0 Gew.-% und besonders bevorzugt von 0,5 bis 2,5 Gew.-% Ammoniak - bezogen auf die Menge Ammoniak (NH₃) in der Gesamtmenge der Zubereitung (A) - eingesetzt werden.

Bei der Zubereitung (B) handelt es sich um die Komponente, welche das Oxidationsmittel enthält.

Erfindungsgemäße Zubereitungen (B) sind dadurch gekennzeichnet, dass sie in einem kosmetischen Träger Wasserstoffperoxid (b1), spezielle langkettige Fettalkohole (b2), Kohlenwasserstoffe (b3) und (b4) ethoxylierte Fettalkohole der Formel (I) enthalten. Im Zuge der zu dieser Erfindung führenden Arbeiten hat sich herausgestellt, dass sich die Kombination aus speziellen langkettigen Fettalkoholen (b2), Kohlenwasserstoffen (b3) und (b4) ethoxylierten Fettalkoholen der Formel (I) hervorragend eignet, die geruchliche Wahrnehmung des Ammoniak während der Anwendung zu unterdrücken.

Überraschenderweise wurde hierbei gefunden, dass die Geruchsunterdrückung am effektivsten ist, wenn die Kombination aus Fettalkoholen (b2), Kohlenwasserstoffen (b3) und ethoxylierten Fettalkoholen der Formel (I) (b4) sich nicht in der Färbecreme (d.h. der Zubereitung (A)), sondern in der das Oxidationsmittel beinhaltenden Zubereitung (B) befindet.

Dass der Ammoniak-Geruch stärker unterdrückt werden kann, wenn Ammoniak (a2) und Fettalkohole (b2) / Kohlenwasserstoffe (b3) / ethoxylierte Fettalkohole der Formel (I) (b4) getrennt gelagert und erst kurz vor der Anwendung miteinander vermischt werden, war für den Fachmann nicht vorhersehbar. Eine Erklärungsmöglichkeit für diesen unerwarteten Effekt könnte eine spezielle, temporäre Emulsionform sein, die sich kurz nach dem Mischungsvorgang zwischen Ammoniak und den Komponenten (b2) / (b3) / (b4) bildet.

Als ersten wesentlichen Inhaltstoff enthält die Zubereitung (B) Wasserstoffperoxid (b1). In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte Zubereitungen (B) sind dadurch gekennzeichnet, dass sie 0,5 bis 20 Gew.-%, vorzugsweise 1 bis 12,5 Gew.-%, besonders bevorzugt 2,5 bis 10 Gew.-% und insbesondere 3 bis 9 Gew.-% Wasserstoffperoxid, jeweils bezogen auf das Gesamtgewicht der Zubereitung (B), enthalten.

Als zweiten erfindungswesentlichen Inhaltsstoff enthält die Zubereitung (B) mindestens einen Fettalkohol (b2) aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol).

Diese langkettigen Fettalkohole (b2) besitzen ein Kettenlänge von mindestens 20 C-Atomen. Innerhalb dieser Gruppe haben sich spezielle langkettige Fettalkohole im Hinblick auf die geruchliche Optimierung der Anwendungsmischungen als ganz besonders geeignet erwiesen.

In einer besonders bevorzugten Ausführungsform ist die Zubereitung (B) dadurch gekennzeichnet, dass sie Arachylalkohol (Eicosan-1-ol) enthält.

In einer weiteren besonders bevorzugten Ausführungsform ist die Zubereitung (B) dadurch gekennzeichnet, dass sie Behenylalkohol (Docosan-1-ol) enthält.

In einer weiteren besonders bevorzugten Ausführungsform ist die Zubereitung (B) dadurch gekennzeichnet, dass sie Arachylalkohol (Eicosan-1-ol) und/oder Behenylalkohol (Docosan-1-ol) enthält.

Weiterhin hat sich herausgestellt, dass es von Vorteil ist, wenn die langkettigen Fettalkohole (b2), insbesondere Arachylalkohol (Eicosan-1-ol) und/oder Behenylalkohol (Docosan-1-ol), in bestimmten Mengenbereichen in der Zubereitung (B) enthalten sind. Hierbei hat es sich als besonders bevorzugt herausgestellt, wenn die Gesamtmenge der Fettalkohole (b2) ausreichend hoch gewählt wird, so dass eine effektive Reduktion des Ammoniakgeruchs erzielt werden kann. Auf der anderen Seite haben sich zu hohe Einsatzmengen an Fettalkoholen (b2) als nachteilig erwiesen, da das Färbemittel in letzterem Fall zu stark verdickt wird. Bedingt durch die zu hohe Viskosität wird die Diffusion der aktiven Spezies (Oxidationsmittel) in den Haarschaft hinein verhindert, was in einer Verschlechterung der Färbeleistung resultiert.

Auf Basis dieser Befunde hat es sich als besonders vorteilhaft herausgestellt, wenn die Zubereitung (B) ein oder mehrere langkettige Fettalkohole (b2) aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,2 bis 6,4 Gew.-%, bevorzugt von 0,3 bis 4,4 Gew.-%, weiter bevorzugt von 0,4 bis 2,4 Gew.-% und besonders bevorzugt von 0,5 bis 1,4 Gew.-% - bezogen auf das Gesamtgewicht der Zubereitung (B) - enthält.

In einer ganz besonders bevorzugten Ausführungsform ist eine erfindungsgemäße Mehrkomponenten-Verpackungseinheit (Kit-of-parts) daher dadurch gekennzeichnet, dass die Zubereitung (B) als Fettalkohol(e) (b2) Arachylalkohol (Eicosan-1-ol) und/oder Behenylalkohol (Docosan-1-ol) in einer Gesamtmenge von 0,2 bis 6,4 Gew.-%, bevorzugt von 0,3 bis 4,4 Gew.-%, weiter bevorzugt von 0,4 bis 2,4 Gew.-% und besonders bevorzugt von 0,5 bis 1,4 Gew.-% - bezogen auf das Gesamtgewicht der Zubereitung (B) - enthält.

Neben den speziellen langkettigen Fettalkoholen (b2) mit einer Kettenlänge von mindestens 20 C-Atomen kann die Zubereitung (A) und/oder (B) zusätzlich auch noch weitere, kürzerkettige Fettalkohole mit einer Kettenlänge von 12 bis 18 C-Atomen enthalten. Geeignete kürzerkettige Fettalkohole mit einer gesättigten C₁₂-C₁₈-Alkylkette sind beispielsweise Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol) und Octadecan-1-ol (Octadecylalkohol, Stearylalkohol). Ein geeigneter kürzerkettiger Fettalkohol mit einer ungesättigten C₁₂-C₁₈-Alkylkette ist beispielsweise (9*Z*)-Octadec-9-en-1-ol (Oleylalkohol).

In der Zubereitung (B) können die kürzerkettigen Fettalkholen mit einer Kettenlänge von 12 bis 18 C-Atome in einer Gesamtmenge Menge von 0,1 bis 6,0 Gew.-%, bevorzugt von 0,3 bis 5,0 Gew.-% und besonders bevorzugt von 0,5 bis 4,0 Gew.-% - bezogen auf das Gesamtgewicht der Zubereitung (B) - enthalten sein.

Auch die Zubereitung (A) kann kürzerkettige Fettalkhole mit einer Kettenlänge von 12 bis 18 C-Atomen enthalten. In der Zubereitung (A) können die kürzerkettigen Fettalkholen mit einer Kettenlänge von 12 bis 18 C-Atome in einer Gesamtmenge von 0,1 bis 12,0 Gew.-%, bevorzugt von 0,3 bis 10,0 Gew.-% und besonders bevorzugt von 0,5 bis 8,0 Gew.-% - bezogen auf das Gesamtgewicht der Zubereitung (A) - enthalten sein.

Als dritten erfindungswesentlichen Bestandteil enthält die Zubereitung (B) mindestens einen Kohlenwasserstoff (b3) mit 8 bis 80 C-Atomen. Unter Kohlenwasserstoffen mit 8 bis 80 C-Atomen sind im Sinne der Erfindung Verbindungen zu verstehen, die ausschließlich aus Kohlenstoff und Wasserstoff bestehen.

Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Es hat sich herausgestellt, dass die C-Kettenverteilung der Paraffinöle ebenfalls Einfluss auf die Effektivität der Geruchsreduzierung nimmt. Als besonders geeignet haben sich in diesem Zusammenhang flüssige Paraffinöle (Paraffinum Liquidum und Paraffinium Perliquidum) erwiesen. Ganz besonders bevorzugt handelt es sich bei dem Kohlenwasserstoff (b3) um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Bevorzugt werden die Kohlenwasserstoffe (b3) mit 8 bis 80 C-Atomen in bestimmten Mengenbereichen eingesetzt. In einer weiteren bevorzugten Ausführungsform enthält die Zubereitung (B) den oder die Kohlenwasserstoff(e) mit 8 bis 80 C-Atomen (b3) in einer Gesamtmenge von 0,1 bis 4,5 Gew.-%, bevorzugt von 0,2 bis 3,2 Gew.-%, weiter bevorzugt von 0,3 bis 1,8 Gew.-% und besonders bevorzugt von 0,4 bis 0,9 Gew.-% - bezogen auf das Gesamtgewicht der Zubereitung (B).

Eine ganz besonders bevorzugte Mehrkomponenten-Verpackungseinheit (Kit-of-parts) ist dadurch gekennzeichnet, dass die Zubereitung (B) als Kohlenwasserstoff(e) (b3) ein oder mehrere Paraffinöle und/oder ein oder mehrere Paraffinwachse in einer Gesamtmenge von 0,1 bis 4,5 Gew.-%, bevorzugt von 0,2 bis 3,2 Gew.-%, weiter bevorzugt von 0,3 bis 1,8 Gew.-% und besonders bevorzugt von 0,4 bis 0,9 Gew.-% - bezogen auf das Gesamtgewicht der Zubereitung (B) - enthält.

Die im Rahmen dieser Erfindung durchgeführten Versuche haben gezeigt, dass eine Wechselwirkung zwischen den langkettigen Fettalkoholen (b2) und den Kohlenwasserstoffen (b3) besteht.

Der Einsatz der langkettigen Fettalkohole (b2) ohne die Kohlenwasserstoffe (insbesondere ohne Paraffinium Liquidum) führte zu einer weniger zufrieden stellenden Unterdrückung des Ammoniak-Geruchs. Wurden beide Komponenten (b2) und (b3) zusammen eingesetzt, war die Geruchswahrnehmung deutlich reduziert. Hierbei konnte der Ammoniakgeruch insbesondere dann reduziert werden, wenn die Komponenten (b2) und (b3) in bestimmten, aufeinander abgestimmten Mengenverhältnissen eingesetzt wurden. Ein Gewichtsverhältnis (b2)/(b3) von 15:1 bis 1:1, bevorzugt von 10:1 bis 1:1, weiter bevorzugt von 7:1 bis 1:1 und besonders bevorzugt von 3:1 bis 2:1 hat in diesem Zusammenhang zu den besten Ergebenissen geführt.

Somit wurde die erfindungsgemäße Aufgabenstellung insbesondere dann gut gelöst, wenn die langkettigen Fettalkhole (b2) im Vergleich zu den Kohlenwasserstoffen (b3) in einem Überschuss eingesetzt wurden.

Aus diesem Grund ist in eine weitere ganz besonders bevorzugte Mehrkomponenten-Verpackungseinheit (Kit-of-parts) dadurch gekennzeichnet, dass die Zubereitung (B) den oder die Fettalkohole (b2) und den oder die aliphatischen Kohlenwasserstoffe (b3) in einem Gewichtsverhältnis (b2)/(b3) von 15:1 bis 1:1, bevorzugt von 10:1 bis 1:1, weiter bevorzugt von 7:1 bis 1:1 und besonders bevorzugt von 3:1 bis 2:1 enthält.

Die in diesem Zusammenhang genannten Gewichtsverhältnisse (b2)/(b3) beziehen sich hierbei jeweils auf die Gesamtmenge aller in der Zubereitung (B) befindlichen langkettigen Fettalkhole (b2), die zu der Gesamtmenge aller in der Zubereitung (B) befindlichen Kohlenwasserstoffe (b3) in Gewichtsrelation gesetzt werden.

Weiterhin hat sich gezeigt, dass sich die Zubereitung (B) durch den Einsatz von zusätzlichen speziellen Inhaltsstoffen im Hinblick auf den Rückhalt des Ammoniak-Geruchs noch weiter optimieren lässt. Vor allem hochethoxylierte Fettalkohole haben sich in dieser Hinsicht als sehr effektiv erwiesen. Enthält die Zubereitung (B) zusätzlich noch mindestens einen ethoxylierten Fettalkohol (b4) mit einem Ethoxylierungsgrad von 80 bis 120, so bleibt die Geruchsminimierung über einen besonders langen Zeitraum signifikant warhnehmbar.

Eine erfindungsgemäße Mehrkomponenten-Verpackungseinheit ist daher dadurch gekennzeichnet, dass die Zubereitung (B) zusätzlich (b4) mindestens einen ethoxylierten Fettalkohol der Formel (I) enthält, 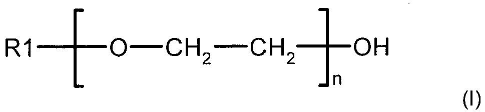 worin
R1 für eine unverzweigte oder verzweigte, gesättige oder ungesättigte C₁₂-C₂₈-Alkylgruppe, bevorzugt für eine gesättigte, unverzweigte C₁₆- oder C₁₈- Alkylgruppe, steht und
n für eine ganze Zahl von 80 bis 120, bevorzugt für eine ganze Zahl von 85 bis 115, weiter bevorzugt für eine ganze Zahl von 90 bis 110 und besonders bevorzugt für eine ganze Zahl von 95 bis 105 steht.

Vorteilhaft ist insbesondere der Zusatz von ethoxylierten Fettalkholen der Formel (I), bei welchen n für eine Zahl von 95 bis 110 steht.

Ethoxylierte Fettalkhole der Formel (I), bei welchen R1 für eine gesättigte, lineare C16-Alkylgruppe oder für eine gesättigte, lineare C18-Alkylgruppe steht, sind ebenfalls ganz besonders bevorzugt. Beispielhaft für diese besonders bevorzugten Verträter lässt sich der Stearylalkohol, ethoxyliert mit 100

Ethylenoxid-Einheiten (INCI-Name Steareth-100, CAS-Nr. 9005-00-9) nennen, welcher beispielsweise unter dem Handelsnamen Brij S 100 oder Brij 700 P von der Firma Croda vertrieben wird.

Weiterhin besonders bevorzugt ist daher eine Mehrkomponenten-eine Mehrkomponenten-Verpackungseinheit (Kit-of-parts) zum oxidativen Färben von keratinischen Fasern, umfassend zwei getrennt voneinander verpackte Zubereitungen (A) und (B), wobei
(a) die Zubereitung (A) in einem kosmetischen Träger
(a1) mindestens ein Oxidationsfarbstoffvorprodukt und
(a2) Ammoniak enthält, und
(b) die Zubereitung (B) in einem kosmetischen Träger
(b1) Wasserstoffperoxid
(b2) mindestens einen Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol)
(b3) Paraffinöl und
(b4) mindestens einen ethoxylierten Fettalkohol der Formel (I) enthält, 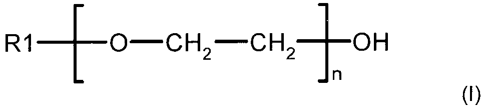 worin
   R1 für eine gesättigte, unverzweigte C₁₆- oder C₁₈- Alkylgruppe, steht und
   n für eine ganze Zahl von 95 bis 105 steht.

Insbesondere bevorzugt ist hierbei eine Mehrkomponenten-eine Mehrkomponenten-Verpackungseinheit (Kit-of-parts) zum oxidativen Färben von keratinischen Fasern, umfassend zwei getrennt voneinander verpackte Zubereitungen (A) und (B), wobei
(a) die Zubereitung (A) in einem kosmetischen Träger
(a1) mindestens ein Oxidationsfarbstoffvorprodukt und
(a2) Ammoniak enthält, und
(b) die Zubereitung (B) in einem kosmetischen Träger
(b1) Wasserstoffperoxid
(b2) mindestens einen Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol)
(b3) Paraffinöl und
(b4) Steareth-100 enthält.

Die ethoxylierten Fettalkhole (b4) der Formel (I) werden vorteilhafterweise in bestimmten Mengenbereichen in der Zubereitung (B) eingesetzt.

Bevorzugte Mehrkomponenten-Verpackungseinheiten zeichnen sich daher dadurch aus, dass die Zubereitung (B) ein oder mehrere ethoxylierte Fettalkohole (b4) der Formel (I) in einer Gesamtmenge von 0,1 bis 5,5 Gew.-%, bevorzugt von 0,2 bis 3,8 Gew.-%, weiter bevorzugt von 0,3 bis 2,6 Gew.-% und besonders bevorzugt von 0,4 bis 1,1 Gew.-% - bezogen auf das Gesamtgewicht der Zubereitung (B) - enthält.

Es hat sich ebenfalls von Vorteil erwiesen, wenn die Zubereitung (A) des Mehrkomponenten-Kits selbst keine ethoxylierten Fettalkhole der Formel (I) enthält, d.h. wenn die Zubereitung (A) von ethoxylierten Fettalkholen der Formel (I) frei ist.

In einer weiteren bevorzugten Ausführungsform ist die Mehrkomponenten-Verpackungseinheit (Kit-of-parts) dadurch gekennzeichnet, dass die Zubereitung (A) frei von ethoxylierten Fettalkohol der Formel (I) ist.

Unter der Definition "frei von ethoxylierten Fettaloholen der Formel (I)" werden in diesem Kontext die Zubereitungen (A) verstanden, die weniger als 0,5 Gew.-%, weiter bevorzugt weniger als 0,4 Gew.-%, noch weiter bevorzugt weniger als 0,1 Gew.-% und besonders bevorzugt weniger als 0,05 Gew.-% an ethoxylierten Fettalkoholen der Formel (I) enthalten. Berechnungsgrundlage für die angegebenen Mengen ist hierbei das Gesamtgewicht der Zubereitung (A).

Bevorzugt ist demzufolge eine Mehrkomponenten-Verpackungseinheit (Kit-of-parts) zum oxidativen Färben von keratinischen Fasern, umfassend zwei getrennt voneinander verpackte Zubereitungen (A) und (B), wobei
(a) die Zubereitung (A) in einem kosmetischen Träger
(a1) mindestens ein Oxidationsfarbstoffvorprodukt und
(a2) Ammoniak enthält,
(a3) frei ist von ethoxylierten Fettalkoholen der Formel (I), 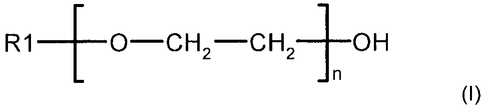 worin
   R1 für eine gesättigte, unverzweigte C₁₆- oder C₁₈- Alkylgruppe, steht und
   n für eine ganze Zahl von 80 bis 120 steht,
(b) die Zubereitung (B) in einem kosmetischen Träger
(b1) Wasserstoffperoxid
(b2) mindestens einen Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol)
(b3) Paraffinöl und
(b4) mindestens einen ethoxylierten Fettalkohol der Formel (I) enthält, 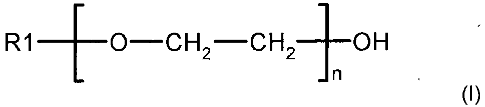 worin
   R1 für eine unverzweigte oder verzweigte, gesättige oder ungesättigte C₁₂-C₂₈-Alkylgruppe, bevorzugt für eine gesättigte, unverzweigte C₁₆- oder C₁₈- Alkylgruppe, steht und
   n für eine ganze Zahl von 80 bis 120, bevorzugt für eine ganze Zahl von 85 bis 115, weiter bevorzugt für eine ganze Zahl von 90 bis 110 und besonders bevorzugt für eine ganze Zahl von 95 bis 105 steht.

Bevorzugt ist demzufolge eine Mehrkomponenten-Verpackungseinheit (Kit-of-parts) zum oxidativen Färben von keratinischen Fasern, umfassend zwei getrennt voneinander verpackte Zubereitungen (A) und (B), wobei
(a) die Zubereitung (A) in einem kosmetischen Träger
(a1) mindestens ein Oxidationsfarbstoffvorprodukt und
(a2) Ammoniak enthält, und
(a3) weniger als 0,1 Gew.-% an ethoxylierten Fettalkoholen der Formel (I) enthält 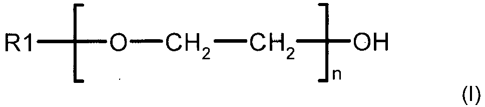 worin
   R1 für eine gesättigte, unverzweigte C₁₆- oder C₁₈- Alkylgruppe, steht und
   n für eine ganze Zahl von 80 bis 120 steht,
(b) die Zubereitung (B) in einem kosmetischen Träger
(b1) Wasserstoffperoxid
(b2) mindestens einen Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol)
(b3) Paraffinöl und
(b4) mindestens einen ethoxylierten Fettalkohol der Formel (I) enthält, 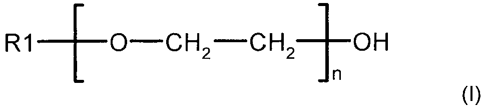 worin
   R1 für eine unverzweigte oder verzweigte, gesättige oder ungesättigte C₁₂-C₂₈-Alkylgruppe, bevorzugt für eine gesättigte, unverzweigte C₁₆- oder C₁₈- Alkylgruppe, steht und
   n für eine ganze Zahl von 80 bis 120, bevorzugt für eine ganze Zahl von 85 bis 115, weiter bevorzugt für eine ganze Zahl von 90 bis 110 und besonders bevorzugt für eine ganze Zahl von 95 bis 105 steht.

Zur Erzielung einer maximalen Geruchsabdeckung werden auch die langkettigen Fettalkohole (b2) und die ethoxylierten Fettalkohole (b4) der Formel (I) in einem optimierten Gewichtsverhältnis zueinander eingesetzt. Von Vorteil es in diesem Zusammenhang, wenn der Fettalkohol (b2) in größerer Menge als der ethoxylierte Fettalkhol (b4) eingesetzt wird.

Wird dagegen der ethoxylierte Fettalkohol (b4) im Vergleich zum langkettigen Fettalkohol (b2) im Überschuss eingesetzt, so ist wieder eine Verschlechterung der geruchlichen Abdeckung zu beobachten.

Aus diesem Grund ist eine weitere besonders bevorzugte Mehrkomponenten-Verpackungseinheit dadurch gekennzeichnet, dass die Zusammensetzung (B) den oder die Fettalkohole (b2) und den oder die ethoxylierten Fettalkohole (b4) der Formel (I) in einem Gewichtsverhältnis (b2)/(b4) von 10:1 bis 1:1, bevorzugt von 5:1 bis 1:1, weiter bevorzugt von 4:1 bis 1:1 und besonders bevorzugt von 3:1 bis 2:1 enthält.

Das Gewichtsverhältnis (b2)/(b4) beziffert hierbei das Gewichtsverhältnis aus der Gesamtmenge aller in der Formulierung (B) enthaltenen langkettigen Fettalkohole (b2) zu der Gesamtmenge aller in der Formulierung (B) enthaltenen ethoxylierten Fettalkohole (b4) der Formel (I).

Von besonderem Vorteil zur Lösung der erfindungsgemäßen Aufgabenstellung ist es auch wenn die Kohlenwasserstoffe (b3) und die ethoxylierten Fettalkohole (b4) der Formel (I) in bestimmten Gewichtsverhältnissen zueinander eingesetzt werden.

Damit ist eine weitere besonders bevorzugte Mehrkomponenten-Verpackungseinheit (Kit-of-parts), dadurch gekennzeichnet, dass die Zusammensetzung (B) den oder die aliphatischen Kohlenwasserstoffe (b3) und den oder die ethoxylierten Fettalkohole (b4) in einem Gewichtsverhältnis (b3)/(b4) von 5:1 bis 1:5, bevorzugt von 3:1 bis 1:3, weiter bevorzugt von 2:1 bis 1:2 und besonders bevorzugt von 2:1 bis 1:1 enthält.

In einer weiteren Ausführungsformen ist eine Mehrkomponenten-Verpackungseinheit besonders bevorzugt, umfassend zwei getrennt voneinander verpackte Zubereitungen (A) und (B),wobei
(a) die Zubereitung (A) in einem kosmetischen Träger
(a1) mindestens ein Oxidationsfarbstoffvorprodukt und
(a2) Ammoniak enthält, und
(b) die Zubereitung (B) in einem kosmetischen Träger
(b1) Wasserstoffperoxid
(b2) mindestens einen Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol),
(b3) mindestens einen Kohlenwasserstoff mit 8 bis 80 C-Atomen und
(b4) mindestens einen ethoxylierten Fettalkohol der Formel (I) enthält, 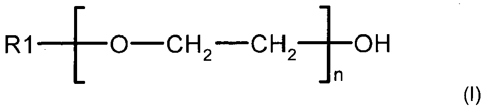 worin
   R1 für eine unverzweigte oder verzweigte, gesättige oder ungesättigte C₁₂-C₂₈-Alkylgruppe, bevorzugt für eine gesättigte, unverzweigte C₁₆- oder C₁₈- Alkylgruppe, steht und
   n für eine ganze Zahl von 80 bis 120, bevorzugt für eine ganze Zahl von 85 bis 115, weiter bevorzugt für eine ganze Zahl von 90 bis 110 und besonders bevorzugt für eine ganze Zahl von 95 bis 105 steht, und die Zubereitung (B) den oder die Fettalkohole (b2) und den oder die Kohlenwasserstoffe (b3) in einem Gewichtsverhältnis (b2)/(b3) von 15:1 bis 1:1, bevorzugt von 10:1 bis 1:1, weiter bevorzugt von 7:1 bis 1:1 und besonders bevorzugt von 3:1 bis 2:1 enthält.

In einer weiteren Ausführungsformen ist eine Mehrkomponenten-Verpackungseinheit besonders bevorzugt, umfassend zwei getrennt voneinander verpackte Zubereitungen (A) und (B), wobei
(a) die Zubereitung (A) in einem kosmetischen Träger
(a1) mindestens ein Oxidationsfarbstoffvorprodukt und
(a2) Ammoniak enthält, und
(b) die Zubereitung (B) in einem kosmetischen Träger
(b1) Wasserstoffperoxid
(b2) mindestens einen Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol),
(b3) mindestens einen Kohlenwasserstoff mit 8 bis 80 C-Atomen und
(b4) mindestens einen ethoxylierten Fettalkohol der Formel (I) enthält, 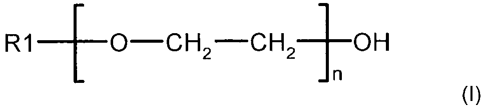 worin
   R1 für eine unverzweigte oder verzweigte, gesättige oder ungesättigte C₁₂-C₂₈-Alkylgruppe, bevorzugt für eine gesättigte, unverzweigte C₁₆- oder C₁₈- Alkylgruppe, steht und
   n für eine ganze Zahl von 80 bis 120, bevorzugt für eine ganze Zahl von 85 bis 115, weiter bevorzugt für eine ganze Zahl von 90 bis 110 und besonders bevorzugt für eine ganze Zahl von 95 bis 105 steht, und die Zusammensetzung (B) den oder die Fettalkohole (b2) und den oder die ethoxylierten Fettalkohole (b4) der Formel (I) in einem Gewichtsverhältnis (b2)/(b4) von 10:1 bis 1:1, bevorzugt von 5:1 bis 1:1, weiter bevorzugt von 4:1 bis 1:1 und besonders bevorzugt von 3:1 bis 2:1 enthält.

In einer weiteren Ausführungsformen ist eine Mehrkomponenten-Verpackungseinheit besonders bevorzugt, umfassend zwei getrennt voneinander verpackte Zubereitungen (A) und (B), wobei
(a) die Zubereitung (A) in einem kosmetischen Träger
(a1) mindestens ein Oxidationsfarbstoffvorprodukt und
(a2) Ammoniak enthält, und
(b) die Zubereitung (B) in einem kosmetischen Träger
(b1) Wasserstoffperoxid
(b2) mindestens einen Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol),
(b3) mindestens einen Kohlenwasserstoff mit 8 bis 80 C-Atomen und
(b4) mindestens einen ethoxylierten Fettalkohol der Formel (I) enthält, 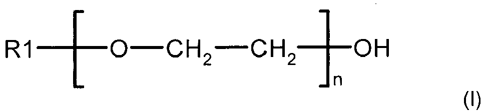 worin
   R1 für eine unverzweigte oder verzweigte, gesättige oder ungesättigte C₁₂-C₂₈-Alkylgruppe, bevorzugt für eine gesättigte, unverzweigte C₁₆- oder C₁₈- Alkylgruppe, steht und
   n für eine ganze Zahl von 80 bis 120, bevorzugt für eine ganze Zahl von 85 bis 115, weiter bevorzugt für eine ganze Zahl von 90 bis 110 und besonders bevorzugt für eine ganze Zahl von 95 bis 105 steht, und die Zusammensetzung (B) den oder die Kohlenwasserstoffe (b3) und den oder die ethoxylierten Fettalkohole (b4) in einem Gewichtsverhältnis (b3)/(b4) von 5:1 bis 1:5, bevorzugt von 3:1 bis 1:3, weiter bevorzugt von 2:1 bis 1:2 und besonders bevorzugt von 2:1 bis 1:1 enthält.

Der in der Zubereitung (A) enthaltene Ammoniak dient sowohl als Alkalisierungsmittel als auch als Quell- und Penetrationsmittel. Nach dem Wissen des Fachmanns hat die Reduktion des Ammoniak-Gehaltes generell eine schlechtere Quellung der Keratinfaser und im Zusammenhang damit auch eine schlechtere Penetrationsfähigkeit der Oxidationsfarbstoffvorprodukte in die Keratinfaser hinein zur Folge. Aus diesem Grund ist der teilweise Ersatz von Ammoniak durch ein anderes Alkalisierungsmittel (wie Alkanolamine, Carbonate oder basische Aminosäuren) im Regelfall mit einem Performanceverlust des Oxidationsfärbemittels verbunden und aus diesem Grund nicht wünschenswert. Es ist daher bevorzugt, wenn die Komponente (A) keine Alkanolamine, keine Carbonatsalze, keine Hydrogencarbonatsalze und keine basischen Aminosäuren enthält.

Eine weitere bevorzugteMehrkomponenten-Verpackungseinheit (Kit-of-parts) ist daher dadurch gekennzeichnet, dass die Zubereitung (A) frei von Alkanolaminen ist.

Unter Alkanolaminen sind aliphatische C₁-C₁₀-Alkylamine zu verstehen, die ein bis drei Hydroxygruppen tragen (wie beispielsweise Hydroxy-C₁-C₁₀-Alkylamine, Di(Hydroxy-C₁-C₅-alkyl)amine oder Tri(hydroxy-C₁-C₃-alkyl)amine). Als Beispiele für Alkanolamine können insbesondere Monoethanolamin, 2-Amino-2-methylpropanol und Triethanolamin genannt werden.

Unter der Definition "frei von Alkanolaminen" werden in diesem Kontext die Zubereitungen (A) verstanden, die weniger als 0,2 Gew.-%, weiter bevorzugt weniger als 0,1 Gew.-%, noch weiter bevorzugt weniger als 0,05 Gew.-% und besonders bevorzugt weniger als 0,01 Gew.-% - bezogen auf das Gesamtgewicht der Zubereitung (A) - an Alkanolaminen enthalten.

Eine weitere bevorzugteMehrkomponenten-Verpackungseinheit (Kit-of-parts) ist dadurch gekennzeichnet, dass die Zubereitung (A) frei von Natriumcarbonat, Natriumhydrogencarbonat, Kaliumcarbonat, Kaliumhydrogencarbonat, Magnesiumcarbonat, Magnesiumhydrogencarbonat, Ammoniumcarbonat und Ammoniumhydrogencarbonat ist.

Unter der Definition "frei von Carbonaten" werden in diesem Kontext die Zubereitungen (A) verstanden, deren Gesamtgehalt an Natriumcarbonat, Natriumhydrogencarbonat, Kaliumcarbonat, Kaliumhydrogencarbonat, Magnesiumcarbonat, Magnesiumhydrogencarbonat, Ammoniumcarbonat und Ammoniumhydrogencarbonat unterhalb von 0,2 Gew.-%, weiter bevorzugt unterhalb von 0,1 Gew.-%, noch weiter bevorzugt unterhalb von 0,05 Gew.-% und besonders bevorzugt unterhalb von 0,01 Gew.-% - bezogen auf das Gesamtgewicht der Zubereitung (A) - liegt.

Eine weitere bevorzugteMehrkomponenten-Verpackungseinheit (Kit-of-parts) ist dadurch gekennzeichnet, dass die Zubereitung (A) frei von den basischen Aminosäuren Arginin, Lysin, Ornithin und Histidin ist.

Unter der Definition "frei von basischen Aminosäuren" werden in diesem Kontext die Zubereitungen (A) verstanden, deren Gesamtgehalt an Arginin, Lysin, Ornithin und Histidin unterhalb von 0,2 Gew.-%, bevorzugt unterhalb von 0,1 Gew.-%, weiter bevorzugt unterhalb von 0,05 Gew.-% und besonders bevorzugt unterhalb von 0,01 Gew.-% - bezogen auf das Gesamtgewicht der Zubereitung (A) - liegt.

In einer weiteren bevorzugten Ausführungsform ist eine erfindungsgemäße Mehrkomponenten-Verpackungseinheit (Kit-of-parts) dadurch gekennzeichnet, dass
- die Zubereitung (A) kein Oxidationsmittel enthält und
- die Zubereitung (B) neben Wasserstoffperoxid kein weiteres Oxidationsmittel enthält.

Die anwendungsbereiten oxidativen Farbänderungsmittel können weiterhin zusätzliche Wirk-, Hilfs- und Zusatzstoffe enthalten, um die Färbe- bzw. Aufhellleistung zu verbessern und weitere gewünschte Eigenschaften der Mittel einzustellen.

Hierbei können die im folgenden genannten weiteren fakultativen Inhaltsstoffe in der Zubereitung (A) und/oder in der Zubereitung (B) der erfindugnsgemäßen Mehrkomponenten-Verpackungseinheit enthalten sein.

Vorzugsweise werden die anwendungsbereiten Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Erfindungsgemäß geeignete Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül.

Erfindungsgemäß geeignete Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß geeignete Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Die anwendungsbereiten Farbveränderungsmittel können auch mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere, kationische, synthetische Polymere, natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen, nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und - distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Kurz vor der Anwendung wird das anwendungsbereite Oxidationsfärbemittel durch Vermischen, (d.h. verschütteln, verrühren oder verquirlen) der beiden Zubereitungen (A) und (B) hergestellt.

Enthält die erfindungsgemäße Mehrkomponenten-Verpackungseinheit mehr als zwei Komponenten, wird die Anwendungsmischung durch Vermischen der Zubereitung (A) und (B) sowie aller gegebenenfalls noch vorhandenen Komponenten (z.B. durch Vermischen der Zubereitungen (A), (B) und (C), oder durch Vermischen der Zubereitungen (A), (B), (C) und (D)) hergestellt. Da der Mischvorgang (d.h. das In-Kontakt-bringen der Oxidationsfarbsotffvorprodukte mit dem Oxdationsmittel) die Farbstoffbildungsreaktion initiiert, wird die Anwendungsformulierung in der Regel nach Möglichkeit direkt nach dem Mischvorgang auf die Keratinfasern appliziert.

In einer bevorzugten Ausführungsform wird die Anwendungsmischung durch das Vermischen von zwei Zubereitungen (A) und (B) hergestellt.

Üblicherweise werden die beiden Zubereitungen (A) und (B) in gleichen Mengen eingsetzt. In diesem Fall ergibt sich die Anwendungsmischung durch Vermischen der Zubereitungen (A) und (B) im Gewichtsverhältnis 1:1. Mischungsverhältnisse (A)/(B) von 3:1 bis 3:1 sind jedoch ebenfalls möglich und erfindungsgemäß.

Ein weiterer Gegenstand der vorliegenden Erfindung ist daher ein Verfahren zum oxidativen Färben von keratinischen Fasern, umfassend die folgenden Schritte in der angegebenen Reihenfolge:
(i) Vermischen von zwei getrennt voneinander verpackten Zubereitungen (A) und (B) einer Mehrkomponenten-Verpackungseinheit des ersten Erfindungsgegenstands zu einem anwendungsbereiten oxidativen Färbemittel,
(ii) Anwendung des oxidativen Färbemittels auf keratinischen Fasern für einen Zeitraum von 1 bis 60 Minuten,
(I) Abspülen des oxidativen Färbemittels.

Eine weitere Ausführungsform ist auch ein Verafhren zum oxidativen Färben von keratinischen Fasern, umfassend die folgenden Schritte in der angegebenen Reihenfolge:
(i) Vermischen von zwei getrennt voneinander verpackten Zubereitungen (A) und (B) einer Mehrkomponenten-Verpackungseinheit des ersten Erfindungsgegenstands im Mischungsverhältnis 3:1 bis 1:3, bevorzugt 2:1 bis 1:2 und besonders bevorzugt im Mischungsverhältnis 1:1,
(ii) Anwendung des oxidativen Färbemittels auf keratinischen Fasern für einen Zeitraum von 1 bis 60 Minuten,
(I) Abspülen des oxidativen Färbemittels.

Bei Vermischen der Färbezubereitung (A) mit der Oxidationsmittelzubereitung (B) bildet sich eine Emulsion aus, bei welcher der Ammoniak geruchlich nur wenig wahrnehmbar ist. Überraschenderweise wurde hierbei für den Mischvorgang eine Zeitabhängigkeit festgestellt. So wurde herausgefunden, dass die Geruchsminimierung dann besonders effektiv ist, wenn die Zubereitung (B), welche die langkettigen Fettalkohole (b2) und die Kohlenwasserstoffe (b3) (sowie gegebenenfalls die ethoxylierten Fettalkohole (b4) der Formel (I)) enthält, mit der Zubereitung (A) für mindestens 5, besser mindestens 10 und noch besser mindestens 15 Sekunden vermischt wird.

Eine besonders bevorzugte Ausführungsform ist daher ein Verfahren zum oxidativen Färben von keratinischen Fasern, welches dadurch gekennzeichnet ist, dass
(i) die Zubereitungen (A) und (B) für einen Zeitraum von 5 bis 60 Sekunden, bevorzugt für einen Zeitraum von 10 bis 50 Sekunden und besonders bevorzugt für einen Zeitraum von 15 bis 40 Sekunden miteinander vermischt werden.

Ein noch längerer Mischvorgang wirkt sich ebenfalls vorteilhaft auf die Geruchsreduktion aus, jedoch wird der Verbraucher aus Gründen des Anwenderkomforts die Zubereitungen (A) und (B) nicht länger als 60 Sekunden vermischen.

Auch wenn gleich nach dem Vermischen die Farbstoffbildungsreaktion beginnt, so ist es hinsichtlich der geruchlichen Eigenschaften der Anwendungsformulierung doch von Vorteil, wenn die zuvor aus (A) und (B) hergestellte Anwendungsformulierung noch für einen Zeitraum von 20 Sekunden bis zu wenigen Minuten in der geschlossenen Applikationsflasche stehen oder ruhen gelassen wird, bevor mit der Applikation begonnen wird.

Ein Zeitraum zwischen dem Ende des Vermischens (i) und dem Beginn der Anwendung (ii), der zwischen 20 Sekunden und 5 Minuten, bevorzugt zwischen 30 Sekunden und 4 Minuten und ganz besonders bevorzugt zwischen 40 Sekunden und 2 Minuten hat sich als der optimale Kompromiss herausgestellt, um sowohl den Ammoniak-Geruch optimal zu überdecken als auch das spätere Farbergebnis nicht nachteilig zu verändern.

Eine ganz besonders bevorzugte Ausführungsform ist daher weiterhin ein erfindungsgemäßes Verfahren, welches dadurch gekennzeichnet ist, dass zwischen dem Vermischen der Zubereitungen (A) und (B) in Schritt (i) und dem Beginn der Anwendung in Schritt (ii) ein Zeitraum von 20 Sekunden bis 5 Minuten, bevorzugt ein Zeitraum von 30 Sekunden bis 4 Minuten und besonders bevorzugt ein Zeitraum von 40 Sekunden bis 2 Minuten liegt.

Die erfindungsgemäßen Mehrkomponenten-Kits des ersten Gegenstands sind oxidative Färbemittel, welche sich dadurch auszeichnen, dass sie bei sehr guter Färbeleistung und hervorragenden Echtheitseigenschaften nur geringen Ammoniak-Geruch verströmen. Der Anwender kann somit die Haare intensiv und langenhaltend färben, nimmt während -der Anwendung jedoch keinen starken Ammoniak-Geruch war.

Bezüglich weiterer bevorzugter Ausführungsformen des erfindungsgemäßen Verfahrens gilt mutatis mutandis das zu den erfindungsgemäßen Mitteln Gesagte.

### Beispiele

### 1. Herstellung der Anwendungsmischungen aus (A) und (B)

Es wurde die folgende Zubereitung (A) hergestellt:

| Zubereitung A, Formulierungsbestandteile | (Gew.-%) |
|---|---|
| Lanette D ^{[1]} | 9,00 |
| Lorol C12-C18 techn. ^{[2]} | 3,00 |
| Texapon NSO ^{[3]} | 11,00 |
| Plantapon LGC Sorb ^{[4]} | 5,00 |
| Eumulgin B1 ^{[5]} | 0,50 |
| Ceteareth-20 ^{[6]} | 0,50 |
| Myristinsäure (Tetradecansäure) | 0,50 |
| Produkt W 37194 ^{[7]} | 2,00 |
| Kaliumhydroxid | 1,05 |
| Natriumsulfit | 0,30 |
| Vitamin C | 0,05 |
| Hydroxyethan-1,1-diphosphonsäure (60 % in Wasser) | 0,20 |
| Ammoniak (25 %ige wässrige Lösung) | 5,80 |
| p-Toluylendiamin, Sulfat | 1,50 |
| Resorcin | 0,60 |
| m-Aminophenol | 0,20 |
| 3-Amino-2-methylamino-6-methoxypyridin | 0,05 |
| Wasser (dest) | ad 100 |

| | |
|---|---|
| [1] C₁₆-C₁₈-Fettalkohol (INCI-Bezeichnung: Cetearyl alcohol) (Cognis) [2] C₁₂-C₁₈-Fettalkohol (INCI-Bezeichnung: Coconut alcohol) (Cognis) [3] Natriumlaurylethersulfat, ethoxyliert mit 2 EO (wässrige Lösung, Gehalt Aktivsubstanz 27 Gew.-%) [4] Laruylglucoside (10-20 Gew.-%), Natriumlaurylglucose Carboxylate (15-25 Gew.-%), Wasser (62 - 67 Gew.-%) [5] C₁₆-C₁₈-Fettalkohol, ethoxyliert (12 EO) (INCI-Bezeichnung: Ceteareth-12) (BASF) [6] C₁₆-C₁₈-Fettalkohol, ethoxyliert (20 EO) [7] N,N,N-Trimethyl-3-[(1-oxo-2-propenyl)amino]-1-propanaminiumchloride, Polymer mit Natrium 2-Propenoat) (INCI: Acrylamidopropyltrimonium chloride /Acrylate Copolymer), 20 Gew.-%ige wässrige Lösung | |

Es wurden die folgenden Zubereitungen B hergestellt:

| Zubereitung B, Formulierungsbestandteile | V (Gew.-%) | E (Gew.-%) |
|---|---|---|
| Cetearylalkohol | 1,80 | 3,70 |
| Behenylalkohol | --- | 1,30 |
| PEG-40 Castor Oil | 0,40 | ---- |
| Paraffinium Liquidum | --- | 0,65 |
| Natriumcetearylsulfat | 0,20 | --- |
| Steareth-100 | --- | 0,60 |
| Ceteareth-30 (C₁₆-C₁₈-Fettalkohol, ethoxyliert (30 EO) | --- | 0,50 |
| Glyceryl Monostearat | --- | 0,50 |
| Hydroxyethan-1,1-diphosphonsäure (60 % in Wasser) | 0,25 | 0,25 |
| Cocoamidopropylbetain (32 %ige Lösung in Wasser) | 0,40 | 0,40 |
| Dinatriumpyrophosphat | 0,20 | 0,20 |
| Wasserstoffperoxid (50 %ige Lösung in Wasser) | 12,0 | 12,0 |
| Wasser (dest.) | ad 100 | ad 100 |

Die Zubereitung (A) und die Zubereitung (B) wurde jeweils im Gewichtsverhältnis 1:1 miteinander für 30 Sekunden in einer geschlossenen Applikationsflasche verschüttelt. Die aus der Zubereitungen (A) und der Vergleichsformulierung (B) hergestellte Zubereitung wurde direkt auf Haaren appliziert. Die aus der Zubereitung (A) und der erfindungsgemäßen Zubereitung (B) hergestellte Anwendungsmischung wurde zunächst für 2 Minuten in der geschlossenen Applikationsflasche stehen gelassen und dann auf Haaren appliziert.

### 2. Bestimmung des Ammoniakqeruchs

Jeweils 10 Minuten nach Beginn des Auftragens der jeweiligen Anwendungsmischungen auf das Haar wurde der Ammoniak-Geruch von jeweils 5 geschulten Personen bewertet. Die Bewertung erfolgte blind, was bedeutet, dass den Personen, die die Bewertung vornahmen, nicht bekannt war, welche Rezeptur sie gerade bewerteten. Aus den Einzelbewertungen wurde jeweils der Mittelwert gebildet.

Der Ammoniak-Geruch wurde auf einer Skala von 0 (quasi kein Geruch wahrnehmbar) bis 10 (sehr starker Ammoniak-Geruch) bewertet.

**Tabelle 4: Ammoniak-Geruch während der Anwendung**

| | A + B (Vergleich) | A + B (erfindungsgemäß) |
|---|---|---|
| Ammoniak-Abdeckung | 5 | 3 |

Es zeigt sich, dass der Ammoniak-Geruch bei Anwendung der erfindungsgemäßen Anwendungsformulierung als deutlich reduziert wahrgenommen wurde.

## Patentansprüche

1. Mehrkomponenten-Verpackungseinheit (Kit-of-parts) zum oxidativen Färben von keratinischen Fasern, umfassend zwei getrennt voneinander verpackte Zubereitungen (A) und (B), wobei
(a) die Zubereitung (A) in einem kosmetischen Träger
(a1) mindestens ein Oxidationsfarbstoffvorprodukt und
(a2) Ammoniak
enthält, und
(b) die Zubereitung (B) in einem kosmetischen Träger
(b1) Wasserstoffperoxid
(b2) mindestens einen Fettalkohol aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und/oder Brassidylalkohol ((13*E*)-Docosen-1-ol),
(b3) mindestens einen Kohlenwasserstoff mit 8 bis 80 C-Atomen und
(b4) mindestens einen ethoxylierten Fettalkohol der Formel (I), 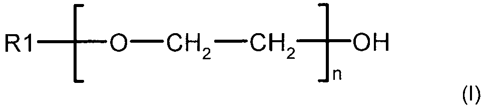 worin
R1 für eine unverzweigte oder verzweigte, gesättigte oder ungesättigte C₁₂-C₂₈-Alkylgruppe steht und
n für eine ganze Zahl von 80 bis 120 steht,
enthält.

2. Mehrkomponenten-Verpackungseinheit (Kit-of-parts) nach Anspruch 1, **dadurch gekennzeichnet, dass** die Zubereitung (B) als Fettalkohol(e) (b2) Arachylalkohol (Eicosan-1-ol) und/oder Behenylalkohol (Docosan-1-ol) in einer Gesamtmenge von 0,2 bis 6,4 Gew.-%, bevorzugt von 0,3 bis 4,4 Gew.-%, weiter bevorzugt von 0,4 bis 2,4 Gew.-% und besonders bevorzugt von 0,5 bis 1,4 Gew.-% - bezogen auf das Gesamtgewicht der Zubereitung (B) - enthält.

3. Mehrkomponenten-Verpackungseinheit (Kit-of-parts) nach einem der Ansprüche 1 bis 2, **dadurch gekennzeichnet, dass** die Zubereitung (B) als Kohlenwasserstoff(e) (b3) ein oder mehrere Paraffinöle und/oder ein oder mehrere Paraffinwachse in einer Gesamtmenge von 0,1 bis 4,5 Gew.-%, bevorzugt von 0,2 bis 3,2 Gew.-%, weiter bevorzugt von 0,3 bis 1,8 Gew.-% und besonders bevorzugt von 0,4 bis 0,9 Gew.-% - bezogen auf das Gesamtgewicht der Zubereitung (B) - enthält.

4. Mehrkomponenten-Verpackungseinheit (Kit-of-parts) nach einem der Ansprüche 1 bis 3, **dadurch gekennzeichnet, dass** die Zubereitung (B) den oder die Fettalkohole (b2) und den oder die Kohlenwasserstoffe (b3) in einem Gewichtsverhältnis (b2)/(b3) von 15:1 bis 1:1, bevorzugt von 10:1 bis 1:1, weiter bevorzugt von 7:1 bis 1:1 und besonders bevorzugt von 3:1 bis 2:1 enthält.

5. Mehrkomponenten-Verpackungseinheit (Kit-of-parts) nach einem der Ansprüche 1 bis 4, **dadurch gekennzeichnet, dass** die Zubereitung (B) zusätzlich
(b4) mindestens einen ethoxylierten Fettalkohol der Formel (I) enthält, 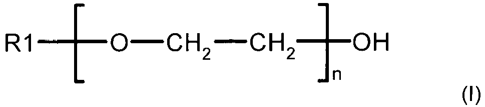 worin
R1 für eine unverzweigte oder verzweigte, gesättigte oder ungesättigte C₁₂-C₂₈-Alkylgruppe, bevorzugt für eine gesättigte, unverzweigte C₁₆- oder C₁₈- Alkylgruppe, steht und
n für eine ganze Zahl von 85 bis 115, weiter bevorzugt für eine ganze Zahl von 90 bis 110 und besonders bevorzugt für eine ganze Zahl von 95 bis 105 steht.

6. Mehrkomponenten-Verpackungseinheit (Kit-of-parts) nach einem der Ansprüche 1 bis 5, **dadurch gekennzeichnet, dass** die Zubereitung (B) ein oder mehrere ethoxylierte Fettalkohole (b4) der Formel (I) in einer Gesamtmenge von 0,1 bis 5,5 Gew.-%, bevorzugt von 0,2 bis 3,8 Gew.-%, weiter bevorzugt von 0,3 bis 2,6 Gew.-% und besonders bevorzugt von 0,4 bis 1,1 Gew.-% - bezogen auf das Gesamtgewicht der Zubereitung (B) - enthält.

7. Mehrkomponenten-Verpackungseinheit (Kit-of-parts) nach einem der Ansprüche 1 bis 6, **dadurch gekennzeichnet, dass** die Zubereitung (A) frei von ethoxylierten Fettalkoholen der Formel (I) ist.

8. Mehrkomponenten-Verpackungseinheit (Kit-of-parts) nach einem der Ansprüche 1 bis 7, **dadurch gekennzeichnet, dass** die Zusammensetzung (B) den oder die Fettalkohole (b2) und den oder die ethoxylierten Fettalkohole (b4) der Formel (I) in einem Gewichtsverhältnis (b2)/(b4) von 10:1 bis 1:1, bevorzugt von 5:1 bis 1:1, weiter bevorzugt von 4:1 bis 1:1 und besonders bevorzugt von 3:1 bis 2:1 enthält.

9. Mehrkomponenten-Verpackungseinheit (Kit-of-parts) nach einem der Ansprüche 1 bis 8, **dadurch gekennzeichnet, dass** die Zusammensetzung (B) den oder die Kohlenwasserstoffe (b3) und den oder die ethoxylierten Fettalkohole (b4) in einem Gewichtsverhältnis (b3)/(b4) von 5:1 bis 1:5, bevorzugt von 3:1 bis 1:3, weiter bevorzugt von 2:1 bis 1:2 und besonders bevorzugt von 2:1 bis 1:1 enthält.

10. Mehrkomponenten-Verpackungseinheit (Kit-of-parts) nach einem der Ansprüche 1 bis 9, **dadurch gekennzeichnet, dass** die Zubereitung (A) frei von Alkanolaminen ist.

11. Mehrkomponenten-Verpackungseinheit (Kit-of-parts) nach einem der Ansprüche 1 bis 10, **dadurch gekennzeichnet, dass**
- die Zubereitung (A) kein Oxidationsmittel enthält und
- die Zubereitung (B) neben Wasserstoffperoxid kein weiteres Oxidationsmittel enthält.

12. Verfahren zum oxidativen Färben von keratinischen Fasern, umfassend die folgenden Schritte in der angegebenen Reihenfolge:
(i) Vermischen von zwei getrennt voneinander verpackten Zubereitungen (A) und (B) einer Mehrkomponenten-Verpackungseinheit nach einem der Ansprüche 1 bis 11 zu einem anwendungsbereiten oxidativen Färbemittel,
(ii) Anwendung des oxidativen Färbemittels auf keratinischen Fasern für einen Zeitraum von 1 bis 60 Minuten,
(iii) Abspülen des oxidativen Färbemittels.

13. Verfahren nach Anspruch 12, **dadurch gekennzeichnet, dass**
(i) die Zubereitungen (A) und (B) für einen Zeitraum von 5 bis 60 Sekunden, bevorzugt für einen Zeitraum von 10 bis 50 Sekunden und besonders bevorzugt für einen Zeitraum von 15 bis 40 Sekunden miteinander vermischt werden.

14. Verfahren nach einem der Ansprüche 12 oder 13, **dadurch gekennzeichnet, dass** zwischen dem Vermischen der Zubereitungen (A) und (B) in Schritt (i) und dem Beginn der Anwendung in Schritt (ii) ein Zeitraum von 20 Sekunden bis 5 Minuten, bevorzugt ein Zeitraum von 30 Sekunden bis 4 Minuten und besonders bevorzugt ein Zeitraum von 40 Sekunden bis 2 Minuten liegt.

## Claims

1. A multicomponent packaging unit (kit of parts) for oxidatively dyeing keratin fibers, comprising two preparations (A) and (B) that are packaged separately from one another, wherein
(a) the preparation (A) contains in a cosmetic carrier
(a1) at least one oxidation dye precursor and
(a2) ammonia
and
(b) the preparation (B) contains in a cosmetic carrier
(b1) hydrogen peroxide
(b2) at least one fatty alcohol from the group arachyl alcohol (eicosan-1-ol), gadoleyl alcohol ((9*Z*)-eicos-9-en-1-ol), arachidonic alcohol ((5*Z*,8*Z*,11*Z*,14*Z*)-eicosa-5,8,11,14-tetraen-1-ol), heneicosyl alcohol (heneicosan-1-ol), behenyl alcohol (docosan-1-ol), erucyl alcohol ((13*Z*)-docos-13-en-1-ol) and/or brassidyl alcohol ((13*E*)-docosen-1-ol),
(b3) at least one hydrocarbon having 8 to 80 C atoms and
(b4) at least one ethoxylated fatty alcohol of formula (I), 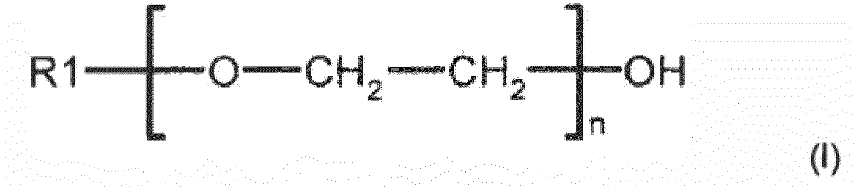 where
R1 represents an unbranched or branched, saturated or unsaturated C₁₂-C₂₈ alkyl group and
n represents an integer from 80 to 120.

2. The multicomponent packaging unit (kit of parts) according to claim 1, **characterized in that** the preparation (B) contains arachyl alcohol (eicosan-1-ol) and/or behenyl alcohol (docosan-1-ol) as the fatty alcohol(s) (b2) in a total amount of from 0.2 to 6.4 wt.%, preferably from 0.3 to 4.4 wt.%, more preferably from 0.4 to 2.4 wt.%, and particularly preferably from 0.5 to 1.4 wt.%, based on the total weight of preparation (B).

3. The multicomponent packaging unit (kit of parts) according to one of claims 1 to 2, **characterized in that** the preparation (B) contains one of more paraffin oils and/or one or more paraffin waxes as the hydrocarbon(s) (b3) in a total amount of from 0.1 to 4.5 wt.%, preferably from 0.2 to 3.2 wt.%, more preferably from 0.3 to 1.8 wt.%, and particularly preferably from 0.4 to 0.9 wt.%, based on the total weight of preparation (B).

4. The multicomponent packaging unit (kit of parts) according to one of claims 1 to 3, **characterized in that** the preparation (B) contains the fatty alcohol(s) (b2) and the hydrocarbon(s) (b3) in a weight ratio (b2)/(b3) of from 15:1 to 1:1, preferably from 10:1 to 1:1, more preferably from 7:1 to 1:1, and particularly preferably from 3:1 to 2:1.

5. The multicomponent packaging unit (kit of parts) according to one of claims 1 to 4, **characterized in that** the preparation (B) additionally contains
(b4) at least one ethoxylated fatty alcohol of formula (I), 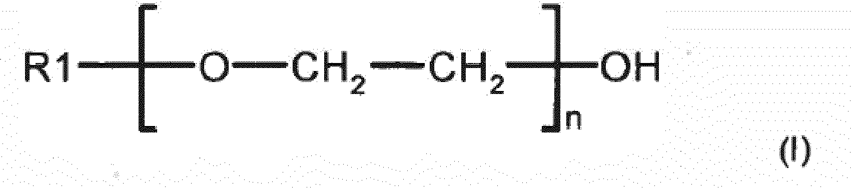 where
R1 represents an unbranched or branched, saturated or unsaturated C₁₂-C₂₈ alkyl group, preferably a saturated, unbranched C₁₆- or C₁₈- alkyl group, and
n represents an integer from 85 to 115, more preferably an integer from 90 to 110, and particularly preferably an integer from 95 to 105.

6. The multicomponent packaging unit (kit of parts) according to one of claims 1 to 5, **characterized in that** the preparation (B) contains one or more ethoxylated fatty alcohols (b4) of formula (I) in a total amount of from 0.1 to 5.5 wt.%, preferably from 0.2 to 3.8 wt.%, more preferably from 0.3 to 2.6 wt.%, and particularly preferably from 0.4 to 1.1 wt.%, based on the total weight of preparation (B).

7. The multicomponent packaging unit (kit of parts) according to one of claims 1 to 6, **characterized in that** the preparation (A) is free of ethoxylated fatty alcohols of formula (I).

8. The multicomponent packaging unit (kit of parts) according to one of claims 1 to 7, **characterized in that** the preparation (B) contains the fatty alcohol(s) (b2) and the ethoxylated fatty alcohol(s) (b4) of formula (I) in a weight ratio (b2)/(b4) of from 10:1 to 1:1, preferably from 5:1 to 1:1, more preferably from 4:1 to 1:1, and particularly preferably from 3:1 to 2:1.

9. The multicomponent packaging unit (kit of parts) according to one of claims 1 to 8, **characterized in that** the preparation (B) contains the hydrocarbon(s) (b3) and the ethoxylated fatty alcohol(s) (b4) in a weight ratio (b3)/(b4) of from 5:1 to 1:5, preferably from 3:1 to 1:3, more preferably from 2:1 to 1:2, and particularly preferably from 2:1 to 1:1.

10. The multicomponent packaging unit (kit of parts) according to one of claims 1 to 9, **characterized in that** the preparation (A) is free of alkanolamines.

11. The multicomponent packaging unit (kit of parts) according to one of claims 1 to 10, **characterized in that**
- the preparation (A) does not contain an oxidizing agent and
- the preparation (B) does not contain a further oxidizing agent in addition to hydrogen peroxide.

12. A method for oxidatively dyeing keratin fibers, comprising the following steps in the stated sequence:
(i) mixing two separately packaged preparations (A) and (B) of a multicomponent packaging unit according to one of claims 1 to 11 to form a ready-to-apply oxidative dye,
(ii) applying the oxidative dye to keratin fibers for a period of from 1 to 60 minutes,
(iii) rinsing out the oxidative dye.

13. The method according to claim 12, **characterized in that**
(i) the preparations (A) and (B) are mixed together for a period of from 5 to 60 seconds, preferably for a period of from 10 to 50 seconds, and particularly preferably for a period of from 15 to 40 seconds.

14. The method according to one of claims 12 or 13, **characterized in that** there is a period of from 20 seconds to 5 minutes, preferably a period of from 30 seconds to 4 minutes, and particularly preferably a period of from 40 seconds to 2 minutes, between mixing the preparations (A) and (B) in step (i) and the start of the application in step (ii).

## Revendications

1. Unité de conditionnement à plusieurs composants (*kit of parts*), destinée à la coloration par oxydation de fibres de kératine, comprenant deux préparations emballées séparément l'une de l'autre (A) et (B), où
(a) la préparation (A) contient, dans un support cosmétique
(a1)au moins un précurseur de colorant d'oxydation, et
(a2)de l'ammoniac, et
(b) la préparation (B) contient, dans un support cosmétique,
(b1)du peroxyde d'hydrogène
(b2)au moins un alcool gras du groupe comprenant l'alcool arachylique (icosan-1-ol), l'alcool gadoléylique ((9Z)-icos-9-én-1-ol), l'alcool arachidonique ((5Z,8Z,11Z,14Z)-icos-5,8,11,14-tétraén-1-ol), l'alcool hénicosylique (hénicosan-1-ol), l'alcool béhénylique (docosan-1-ol), l'alcool érucylique ((13Z)-docos-13-én-1-ol) et/ou l'alcool brassidylique ((13E)-docosén-1-ol),
(b3)au moins un hydrocarbure ayant 8 à 80 atomes de carbone, et
(b4)au moins un alcool gras éthoxylé de formule (I) 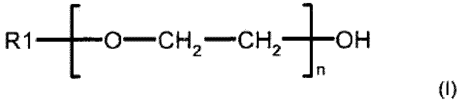 où
R1 représente un groupe C₁₂₋₂₈-alkyle linéaire ou ramifié, saturé ou insaturé, et
n est un entier de 80 à 120.

2. Unité de conditionnement à plusieurs composants (*kit of parts*) selon la revendication 1, **caractérisée en ce que** la préparation (B) contient comme alcool(s) gras (b2) l'alcool arachylique (icosan-1-ol) et/ou l'alcool béhénylique (docosan-1-ol) dans une quantité totale de 0,2 à 6,4 % en poids, de préférence de 0,3 à 4,4 % en poids, plus préférablement de 0,4 à 2,4 % en poids et de manière particulièrement préférée de 0,5 à 1,4 % en poids - rapportée au poids total de la préparation (B).

3. Unité de conditionnement à plusieurs composants (*kit of parts*) selon l'une des revendications 1 à 2, **caractérisée en ce que** la préparation (B) contient comme hydrocarbure(s) (b3) au moins une huile de paraffine et/ou au moins une cire de paraffine dans une quantité totale de 0,1 à 4,5 % en poids, de préférence de 0,2 à 3,2 % en poids, plus préférablement de 0,3 à 1,8 % en poids et de manière particulièrement préférée de 0,4 à 0,9 % en poids - rapportée au poids total de la préparation (B).

4. Unité de conditionnement à plusieurs composants (*kit of parts*) selon l'une des revendications 1 à 3, **caractérisée en ce que** la préparation (B) contient le ou les alcools gras (b2) et le ou les hydrocarbures (b3) dans un rapport pondéral (b2)/(b3) de 15:1 à 1:1, de préférence de 10:1 à 1:1, plus préférablement de 7:1 à 1:1 et de manière particulièrement préférée de 3:1 à 2:1.

5. Unité de conditionnement à plusieurs composants (*kit of parts*) selon l'une des revendications 1 à 4, **caractérisée en ce que** la préparation (B) contient en plus
(b4)au moins un alcool gras éthoxylé de formule (I) 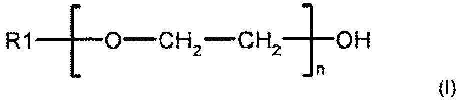 où
R1 représente un groupe C₁₂₋₂₈-alkyle linéaire ou ramifié, saturé ou insaturé, et
n est un entier de 85 à 115, plus préférablement un entier de 90 à 110 et de manière particulièrement préférée un entier de 95 à 105.

6. Unité de conditionnement à plusieurs composants (*kit of parts*) selon une des revendications 1 à 5, **caractérisée en ce que** la préparation (B) contient au moins un alcool gras éthoxylé (b4) de formule (I) dans une quantité totale de 0,1 à 5,5 % en poids, de préférence de 0,2 à 3,8 % en poids, plus préférablement de 0,3 à 2,6 % en poids et de manière particulièrement préférée de 0,4 à 1,1 % en poids - rapportée au poids total de la préparation (B).

7. Unité de conditionnement à plusieurs composants (*kit of parts*) selon l'une des revendications 1 à 6, **caractérisée en ce que** la préparation (A) est exempte d'alcools gras éthoxylés de formule (I).

8. Unité de conditionnement à plusieurs composants (*kit of parts*) selon l'une des revendications 1 à 7, **caractérisée en ce que** la préparation (B) contient l'au moins un alcool gras (b2) et l'au moins un alcool gras (b4) de formule (I) dans un rapport en poids (b2)/(b4) de 10:1 à 1:1, de préférence de 5:1 à 1:1, plus préférablement de 4:1 à 1:1 et de manière particulièrement préférée de 3:1 à 2:1.

9. Unité de conditionnement à plusieurs composants (*kit of parts*) selon l'une des revendications 1 à 8, **caractérisée en ce que** la préparation (B) contient l'au moins un hydrocarbure (b3) et l'au moins un alcool gras (b4) de formule (I) dans un rapport en poids (b3)/(b4) de 5:1 à 1:5, de préférence de 3:1 à 1:3, plus préférablement de 2:1 à 1:2 et de manière particulièrement préférée de 2:1 à 1:1.

10. Unité de conditionnement à plusieurs composants (*kit of parts*) selon l'une des revendications 1 à 9, **caractérisée en ce que** la préparation (A) est exempte d'alcanolamines.

11. Unité de conditionnement à plusieurs composants (*kit of parts*) selon l'une des revendications 1 à 10, **caractérisée en ce que** :
- la préparation (A) ne contient pas d'oxydant, et
- la préparation (B) ne contient, en dehors du peroxyde d'hydrogène, aucun autre oxydant.

12. Procédé de coloration par oxydation de fibres de kératine, comprenant les étapes suivantes dans l'ordre suivant :
(i) mélanger deux préparations emballées séparément l'une de l'autre (A) et (B) d'une unité de conditionnement à plusieurs composants selon l'une des revendications 1 à 11 pour obtenir un agent de coloration par oxydation prêt à l'emploi,
(ii) utiliser l'agent de coloration par oxydation sur des fibres de kératine pendant une durée de 1 à 60 minutes,
(iii) éliminer l'agent de coloration par oxydation par rinçage.

13. Procédé selon la revendication 12, **caractérisé en ce que** :
(i) les préparations (A) et (B) sont mélangées l'une avec l'autre pendant une durée de 5 à 60 secondes, de préférence pendant une durée de 10 à 50 secondes et de manière particulièrement préférée pendant une durée de 15 à 40 secondes.

14. Procédé selon l'une des revendications 12 ou 13, **caractérisé en ce qu'**il s'écoule une durée de 20 secondes à 5 minutes, de préférence une durée de 30 secondes à 4 minutes et de manière particulièrement préférée une durée de 40 secondes à 2 minutes, entre le mélange des préparations (A) et (B) à l'étape (i) et le début de l'utilisation à l'étape (ii).
